# EUROPEAN PATENT SPECIFICATION

(11) **EP 2 892 520 B1**
(45) Date of publication and mention of the grant of the patent: **04.11.2020**
(21) Application number: 13779749.4
(22) Date of filing: 04.09.2013
(51) Int. Cl.: A61K 31/137, A61K 31/138, A61K 31/185, A61K 31/195, A61K 31/197, A61K 31/20, A61K 31/222, A61K 31/4015, A61K 31/4166, A61K 31/4192, A61K 31/515, A61K 31/53, A61K 31/55, A61K 31/5513, A61K 31/635, A61K 31/64, A61K 31/7048, A61P 25/08

(54) **THERAPEUTIC APPROACHES FOR TREATING EPILEPSY AND RELATED DISORDERS THROUGH REDUCTION OF EPILEPTOGENESIS**
THERAPEUTISCHE ANSÄTZE ZUR BEHANDLUNG VON EPILEPSIE UND ZUGEHÖRIGEN ERKRANKUNGEN DURCH REDUZIERUNG DER EPILEPTOGENESE
APPROCHES THÉRAPEUTIQUES POUR LE TRAITEMENT DE L'ÉPILEPSIE ET DES TROUBLES ASSOCIÉS PAR RÉDUCTION DE L'ÉPILEPTOGENÈSE

(30) Priority: 05.09.2012 EP 12306064; 05.09.2012 US 201261697009 P
(43) Date of publication of application: 15.07.2015
(73) Proprietor: Pharnext, 92130 Issy-les-Moulineaux (FR)
(72) Inventor: COHEN, Daniel, 92210 Saint Cloud (FR); NABIROCHKIN, Serguei, 92290 Chatenay Malabry (FR); CHUMAKOV, Ilya, 77000 Vaux le Penil (FR)
(74) Representative: Becker, Philippe
(86) International application number: PCT/EP2013/068313
(87) International publication number: WO 2014/037417

(56) References cited:
- WO-A1-00/20395
- WO-A1-2004/054570
- WO-A1-2007/053596
- WO-A1-2007/112280
- WO-A1-2011/150380
- WO-A2-2009/004082
- WO-A2-2012/117073
- US-A1- 2012 004 225
- US-A1- 2012 115 919
- DATABASE MEDLINE [Online] US NATIONAL LIBRARY OF MEDICINE (NLM), BETHESDA, MD, US; January 2000 (2000-01), ENOKI H ET AL: "[Clinical applications and the effect of mexiletine on refractory epilepsies].", XP002687992, Database accession no. NLM10655748
- "Therapeutic category and Biological Activity Index: ANTICONVULSANT" In: Merck and Co., INC: "THE MERCK INDEX", 2006, Merck Research Laboratories, USA, XP002687993, pages THER-7
- CRAIG A ERICKSON ET AL: "Brief Report: Acamprosate in Fragile X Syndrome", JOURNAL OF AUTISM AND DEVELOPMENTAL DISORDERS, KLUWER ACADEMIC PUBLISHERS-PLENUM PUBLISHERS, NE, vol. 40, no. 11, 6 March 2010 (2010-03-06) , pages 1412-1416, XP019859229, ISSN: 1573-3432, DOI: 10.1007/S10803-010-0988-9

## Description

### FIELD OF THE INVENTION

The present invention relates to compositions for use in the treatment of epilepsy and related disorders. More specifically, the present invention relates to combinatorial therapies of epilepsy and related disorders.

### BACKGROUND OF THE INVENTION

Epilepsy is a chronic neurological disorder characterized by recurrent and unprovoked seizures. According to WHO estimates, around 50 million people are affected by epilepsy across the world and nearly 80% of them are found in developing regions (around 1.9 million patients in Europe and 2.7 million in the United States). Although people of all age groups can be affected by epileptic seizures, the very young and the elderly are more prone to the condition. Based on clinical and electroencephalo graphic (EEG) criteria, seizures are divided into generalized (seizure onset in both hemispheres at once) or partial (focal; seizure onset in one part of the brain). Generalized seizures are subdivided as absence, myoclonic, atonic and tonic-clonic seizures, while partial seizures are further categorized as simple and complex.

It is estimated that the chance of having epilepsy during a lifetime of 80 years is about 3%. In about 30% of cases, there is an identifiable injury to the brain that triggered the development of epilepsy (symptomatic epilepsies). Another 30% of patients have presumed symptomatic epilepsy, in which the cause has not been identified. Brain insults such as traumatic brain injury, stroke, status epilepticus and infection/inflammation are some of the causes of acquired epilepsy, which usually occurs after a latent period and is often progressive (*i.e.* the seizures become more frequent and severe over time). Epileptic seizures may also occur in recovering patients as a consequence of brain surgery. About 1% of all people develops recurrent unprovoked seizures without obvious reason or any other neurological abnormalities. These are named idiopathic epilepsies, generalized or partial, and they are assumed to be mainly of genetic origin [1]. Some genes, coding for protein subunits of voltage-gated and ligand-gated ion channels, have been associated with forms of generalized epilepsy and infantile seizure syndromes. In epilepsy, the brain has become permanently altered pathophysiologically or structurally leading to abnormal, hypersynchronous neuronal firing. Progressive brain damage can occur as a consequence of repeated seizures [2]. For example, a progressive decrease in hippocampal volume over time as a function of seizure number has been reported. Several clinical and experimental data have implicated the failure of blood-brain barrier (BBB) function in triggering chronic or acute seizures [3].

Despite the availability of numerous new antiepileptic drugs (ezogabine, pregabalin, levetiracetam, lamotrigine, topiramate, valproate, rufinamide, gabapentin, carbamazepine, clonazepam, oxcarbazepine, phenobarbital and phenytoin), our ability to eradicate seizures remains limited; approximately one-third of patients still have uncontrolled seizures and even larger percentage suffers from at least one anticonvulsant related side-effect (e.g. mood changes, sleepiness, or unsteadiness in gait) [4]. Furthermore, although seizures represent the most dramatic hallmark of epilepsy, many epilepsy patients develop neurological or psychiatric disease (memory or cognitive impairment, depression...) [5]. For example, mesial temporal lobe epilepsy is usually accompanied by memory deficits probably due to hippocampal system damages and/or brain inflammation [6].

Although the treatment for epilepsy has evolved in the last decade, the present treatment options are not efficacious enough to cure the disease. The existing treatments are focused on preventing seizures once they are underway but none offers a complete cure of the disease. Indeed most anticonvulsants are able to block kindled seizure expression without affecting kindling development [7]. Epilepsy surgery is proposed to intractable patients after confirmation of diagnosis in order to control seizures as well as improve quality-of-life [8]. However, we need the identification of new drug molecules to prevent epilepsy development. Epilepsy seems to be a progressive disease; epileptogenesis refers to a dynamic process that progressively alters neuronal excitability and by which a normal brain develops epilepsy [9]. The term "epileptogenesis" can be expanded to include the possibly never-ending process of clinical progression that involves changes in seizure frequency and the development of a refractory state (pharmacoresistant patients). Many human patients with acquired epilepsy clearly show an increase in seizure frequency and/or severity over time, particularly when considered over several decades [10].

Despite studies on the epileptogenesis process, epilepsy remains so far an incurable disease and no effective disease-modifying treatment has been discovered yet [11]. Current treatments aim at relieving symptoms and slowing the irreversible progression of the disease. Hence, there is a strong unmet need for efficient therapies averting the epileptogenesis and pharmacoresistance along with the long term physiological, behavioral and cognitive consequences of epilepsy.

WO2011/150380 discloses prodrugs of acamprosate of Formula (I) and their use in the treatment of various pathologies such as Fragile X syndrome, autism, Down's syndrome, a neurological disorder and/ or mental retardation in order to diminish, halt, ameliorate or prevent one or more of the neurological deficiencies or symptoms associated with the disorder. Epilepsy is mentioned among these pathologies.

### SUMMARY OF INVENTION

The purpose of the present invention is to provide a composition comprising at least acamprosate, or a salt or sustained release formulation thereof, for use in the treatment of epilepsy, benign Rolandic epilepsy, frontal lobe epilepsy, infantile spasms, myoclonic epilepsy, absence epilepsy, Lennox-Gastaut syndrome, Landau-Kleffner syndrome, Dravet syndrome, progressive myoclonus epilepsies, reflex epilepsy, Rasmussen's syndrome, temporal lobe epilepsy, limbic epilepsy, status epilepticus, abdominal epilepsy, massive bilateral myoclonus, catamenial epilepsy, Jacksonian seizure disorder, Lafora disease or photosensitive epilepsy in a subject in need thereof.

The composition can further comprise at least one compound selected from baclofen, cinacalcet, mexiletine, sulfisoxazole or torasemide, or a salt or sustained release formulation thereof. In particular, the present invention proposes compositions using a drug or a drug combination, as defined in the claims, that inhibits epileptogenesis.

The invention stems, *inter alia,* from the unexpected discovery, by the inventors, that compositions comprising acamprosate provide substantial benefits to patients with epilepsy. Moreover, the inventors have surprisingly discovered that acamprosate and its drug combinations provide strong effect on biological processes involved in epileptogenesis and epilepsy.

More particularly, the invention relates to compositions comprising acamprosate and additionally at least one compound selected from the group consisting of baclofen, cinacalcet, mexiletine, sulfisoxazole and torasemide, or a salt, or sustained release formulation thereof, for use in the treatment of epilepsy or related disorders, in a subject in need thereof.

A further object of the invention resides in a combination of acamprosate, and additionally at least one compound selected from the group consisting of baclofen, cinacalcet, mexiletine, sulfisoxazole and torasemide, or a salt, or sustained release formulation thereof, for use in the treatment of epilepsy or a related disorder in a subject in need thereof by simultaneous, separate, sequential and/or repeated administration to the subject.

A further object of the invention relates to compositions comprising the following combination of compounds: - baclofen and acamprosate.

The compositions may comprise one or several additional active ingredient(s). In this regard, a further object of this invention relates to a composition comprising a combination of acamprosate and at least one compound selected from the group consisting of baclofen, cinacalcet, mexiletine, sulfisoxazole and torasemide, and at least one other compound selected from the group consisting of ezogabine, pregabalin, levetiracetam, lamotrigine, topiramate, valproate, rufinamide, gabapentin, carbamazepine, clonazepam, oxcarbazepine, phenobarbital and phenytoin, or salts or sustained release formulations thereof.

The disclosure also relates to any pharmaceutical composition *per se* comprising a combination of at least two compounds or at least three compounds as defined above.

As it will be further disclosed in the present application, the compounds in a combinatorial therapy of the invention may be administered simultaneously, separately, sequentially and/or repeatedly to the subject.

The compositions of the invention typically further comprise one or several pharmaceutically acceptable excipients or carriers. Also, the compounds as used in the present invention may be in the form of a salt. They may also be in the form of sustained-release formulations.

Also disclosed is a method for treating epilepsy or a related disorder in a mammalian subject in need thereof, preferably a human subject in need thereof, the method comprising administering to said subject an effective amount of a combination of the invention as disclosed above.

A preferred object of this invention relates to acamprosate for use in a method of treating epilepsy or a related disorder in a mammalian subject in need thereof, preferably a human subject in need thereof, the method comprising simultaneously, separately or sequentially administering to said subject, a composition comprising acamprosate and at least one additional compound selected from the group consisting of baclofen, cinacalcet, mexiletine, sulfisoxazole and torasemide, or a salt, or sustained release formulation thereof.

A more preferred object of this invention relates to acamprosate for use in a method of treating epilepsy or a related disorder in a mammalian subject in need thereof, preferably a human subject in need thereof, the method comprising simultaneously, separately or sequentially administering to said subject an effective amount of the following combination of compounds:
- baclofen and acamprosate.

Another object of the invention relates to a composition as disclosed above for use in the treatment of epilepsy or related disorders in a subject who has undergone or will undergo surgical therapy for epilepsy.

Another object of the invention relates to a composition as disclosed above for use in the treatment of epilepsy comorbidities in a subject in need thereof.

Another object of the invention relates to a composition as disclosed above for use in the treatment of epilepsy comorbidities in a subject is at risk of developing epilepsy or a related disorder.

The present invention is defined in the appended claims. Subject matters which are not encompassed by the scope of the claims do not form part of the present invention.

### BRIEF DESCRIPTION OF THE FIGURES

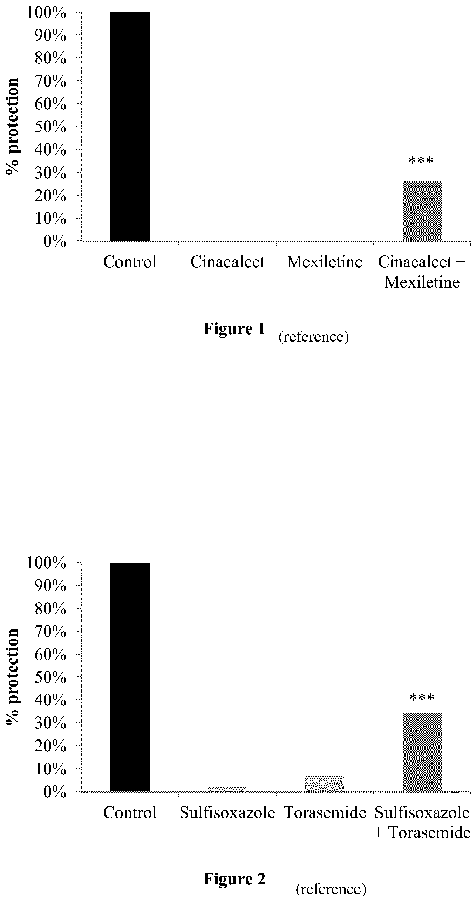

**Figure 1** (reference) : Protection provided by the cinacalcet and mexiletine combination therapy against glutamate toxicity on neuronal cortical cells. The glutamate intoxication is significantly prevented (26% of protection) by the combination of cinacalcet (64 pM) and mexiletine (25.6 pM) whereas, at those concentrations, cinacalcet and mexiletine alone have no significant effect on intoxication. ***: p < 0.001, significantly different from glutamate intoxication (ANOVA + Dunnett Post-Hoc test).
**Figure 2** (reference) : Protection provided by the sulfisoxazole and torasemide combination therapy against glutamate toxicity on neuronal cortical cells. The glutamate intoxication is significantly prevented (34% of protection) by the combination of sulfisoxazole (6.8 nM) and torasemide (400 nM) whereas, at those concentrations, sulfisoxazole and torasemide alone have no significant effect on intoxication. ***: p < 0.001, significantly different from glutamate intoxication (ANOVA + Dunnett Post-Hoc test).

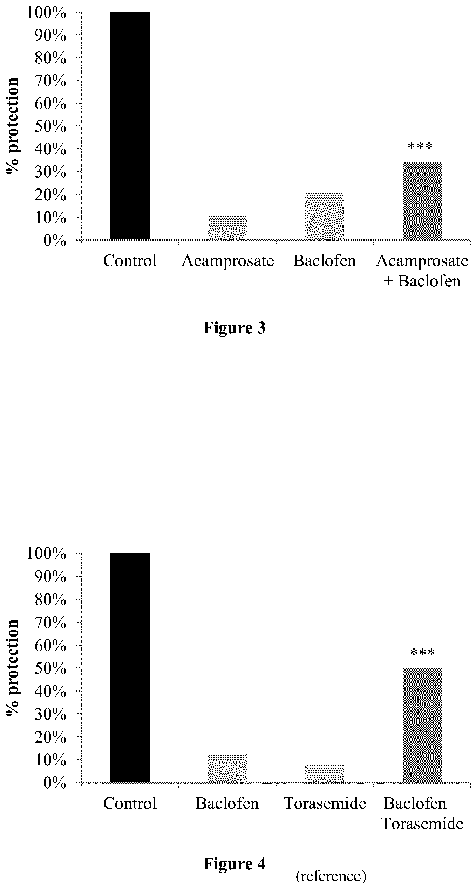

**Figure 3****:** Protection provided by the baclofen and acamprosate combination therapy against glutamate toxicity on neuronal cortical cells. The glutamate intoxication is significantly prevented (34% of protection) by the combination of baclofen (400 nM) and acamprosate (1.6 nM) whereas, at those concentrations, baclofen and acamprosate alone have no statistically significant effect on intoxication. ***: p < 0.001, significantly different from glutamate intoxication (ANOVA + Dunnett Post-Hoc test).
**Figure 4** (reference) : Protection provided by the baclofen and torasemide combination therapy against glutamate toxicity on neuronal cortical cells. The glutamate intoxication is significantly prevented (50% of protection) by the combination of baclofen (2 µM) and torasemide (400 nM) whereas, at those concentrations, baclofen and torasemide alone have no statistically significant effect on intoxication. ***: p < 0.001, significantly different from glutamate intoxication (ANOVA + Dunnett Post-Hoc test).

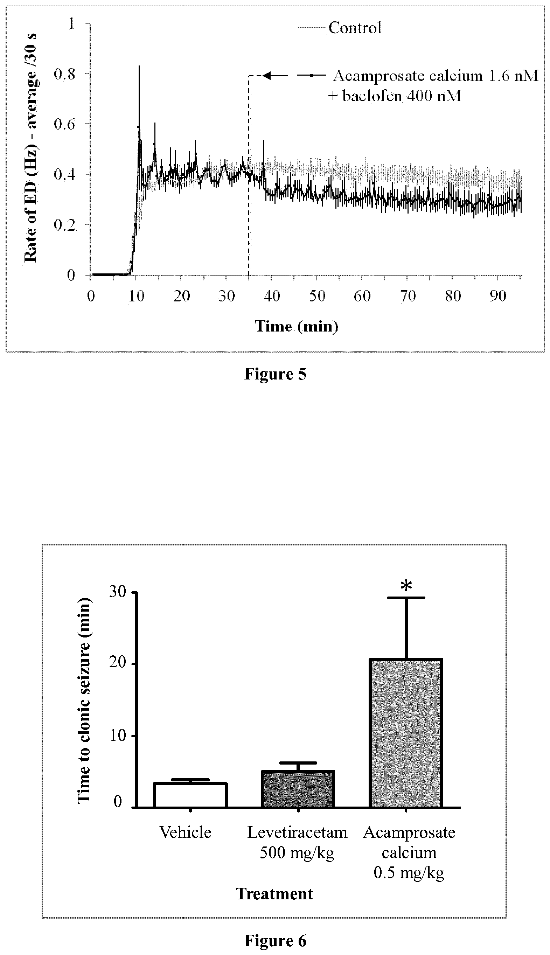

**Figure 5****:** Rate of Epileptiform Discharges averaged by 30 s (ED, ± s.e.m.) all along the experiment (min) in rat hippocampal slices. The combination of acamprosate calcium and baclofen (final concentration of 1.6 nM and 400 nM, respectively, black line) was added at 35 min and was efficient in reducing the rate of ED compared to the control (*i.e.* not treated slices, grey line).
**Figure 6****:** Mean time to clonic seizure (min, + s.e.m.) according to the treatment (vehicle, white bar: water; levetiracetam, dark grey bar: 500 mg/kg; acamprosate calcium, light grey bar: 0.5 mg/kg). Acamprosate calcium is efficient in increasing the latency of clonic seizure in PTZ challenge on 120CD-1 mice model. *: p < 0.05, significantly different from vehicle treated animals.

### DETAILED DESCRIPTION OF THE INVENTION

Epilepsy, in the context of the invention refers to symptomatic epilepsies (identifiable injury to the brain that has triggered the development of epilepsy) or idiopathic epilepsies (mainly of genetic origin). Epilepsy is characterized by recurrent unprovoked seizures. However, it also involves subtle physiological and structural modifications of the neuronal network, often designated under the term epileptogenesis. It is the process by which the brain becomes epileptic, involving changes that occur at various levels to induce a permanent state of hyperexcitability of neurons and hypersynchrony of neuronal networks.

Epileptogenesis is regulated by multiple molecular pathways such as signal transduction, protein transport and processing, regulation of cell death or cell damage. Accordingly, the inventors discovered drugs which can, in combination, effectively modulate epileptogenesis through interactions with these essential molecular pathways.

Hence, the invention relates to the use of combinations of particular drugs which, in combinations, modulate the above pathways and may be used to treat said epilepsy and related disorders.

In a particular embodiment, the present invention more specifically relates to compositions using a drug combination that inhibits epileptogenesis. Thus, therapeutic approaches of the invention are effective for the prevention of epileptic seizures, the reduction of disabling comorbidities associated with epilepsy (such as memory impairment, depression or anxiety), the modulation of the process leading to pharmacoresistance and thus the enhancement of the therapeutic effect of anticonvulsant drugs.

The invention is particularly suited for treating epilepsy and related disorders. In the context of this invention, the term "related disorder" includes benign Rolandic epilepsy, frontal lobe epilepsy, infantile spasms, myoclonic epilepsy, absence epilepsy, Lennox-Gastaut syndrome, Landau-Kleffner syndrome, Dravet syndrome, progressive myoclonus epilepsies, reflex epilepsy, Rasmussen's syndrome, temporal lobe epilepsy, limbic epilepsy, status epilepticus, abdominal epilepsy, massive bilateral myoclonus, catamenial epilepsy, Jacksonian seizure disorder, Lafora disease or photosensitive epilepsy.

As used herein, "treatment" includes the therapy, prevention, prophylaxis, retardation or reduction of symptoms provoked by or of the causes of epilepsy. The term treatment includes in particular the control of disease progression and associated symptoms.

The term "combination or combinatorial treating/therapy" designates a treatment wherein at least two or more drugs are co-administered to a subject to cause a biological effect. In a combined therapy according to this invention, the at least two drugs may be administered together or separately, at the same time or sequentially. Also, the at least two drugs may be administered through different routes and protocols. As a result, although they may be formulated together, the drugs of a combination may also be formulated separately.

The term "salt" refers to a pharmaceutically acceptable and relatively non-toxic, inorganic or organic acid addition salt of a compound of the present invention. Pharmaceutical salt formation consists in pairing an acidic, basic or zwitterionic drug molecule with a counterion to create a salt version of the drug. A wide variety of chemical species can be used in neutralization reaction. Pharmaceutically acceptable salts of the invention thus include those obtained by reacting the main compound, functioning as a base, with an inorganic or organic acid to form a salt, for example, salts of acetic acid, nitric acid, tartric acid, hydrochloric acid, sulfuric acid, phosphoric acid, methane sulfonic acid, camphor sulfonic acid, oxalic acid, maleic acid, succinic acid or citric acid. Pharmaceutically acceptable salts of the invention also include those in which the main compound functions as an acid and is reacted with an appropriate base to form, *e.g.,* sodium, potassium, calcium, magnesium, ammonium, or choline salts. Though most of salts of a given active principle are bioequivalents, some may have, among others, increased solubility or bioavailability properties. Salt selection is now a common standard operation in the process of drug development as teached by H. Stahl and C.G Wermuth in their handbook [28]. A particularly preferred salt for use in the present invention is acamprosate calcium that corresponds to a half of a calcium ion with one molecule of acamprosate.

The term "epilepsy comorbidities" refers to anxiety, suicidal thoughts, depression, memory impairment and/or cognitive decline.

In a more preferred embodiment, the designation of a compound is meant to designate the compound as specifically designated *per se,* as well as any pharmaceutically acceptable salt thereof.

In a particular embodiment, a sustained-release formulation of the compound is used.

As disclosed in the examples, drug combinations of the invention have a strong effect on biological processes involved in epileptogenesis and epilepsy. They represent new therapeutic approaches of the pathology. In particular, compositions of the invention have an effect on the glutamate excitoxicity which is a component of the pathology, but also on the frequency and the severity of convulsing seizures and thus represent new therapeutic approaches of epilepsy. Furthermore, drug combinations of the invention, *in vivo,* induce a protective effect on cognitive functions and behaviour such as short term and long term memory. Thus, new therapeutic approaches of the invention are effective for the protection against epilepsy comorbidities particularly for the protection of memory in a subject suffering from epilepsy. At last, prevention or reduction of epileptogenesis can enhance or maintains the efficacy of anticonvulsants and thus combinations of the invention used in further combination with anticonvulsants are particularly efficient therapies.

Considering the plurality of molecular pathways involved in epileptogenesis, and particularly in epilepsy, drug combinations are particularly advantageous, even if drugs of the invention, when used alone, show any activity in epilepsy experimental models. Indeed, as exemplified in the experimental section drugs of the invention alone or in combination are efficient in protecting neuronal cell from glutamate toxicity, or in delaying time to seizure triggering. In this regard, in a particular embodiment, the present invention more specifically relates to compositions using a drug combination that prevent or reduce epileptogenesis and can be used for the treatment of epilepsy.

In this regard, a preferred object of this invention relates to a composition comprising a combination of acamprosate and at least one compound selected from the group consisting of baclofen, cinacalcet, mexiletine, sulfisoxazole and torasemide, or salts thereof, for use in the treatment of epilepsy or related disorders.

Illustrative CAS numbers for the above cited drugs are provided in table 1 below. Table 1 cites also common salts, racemates, prodrugs, metabolites or derivatives for these compounds used in the compositions of the invention.

**Table 1**

| *Drug* | CAS Numbers | Class or Tanimoto similarity index |
|---|---|---|
| *Acamprosate and related compounds* | | |
| *Acamprosate* | 77337-76-9 ; 77337-73-6 | NA |
| *Homotaurine* | 3687-18-1 | 0.73 |
| *Ethyl dimethyl ammonio propane sulfonate* | / | 0.77 |
| *Taurine* | 107-35-7 | 0.5 |

| *Baclofen and related compounds* | | |
|---|---|---|
| *Baclofen* | 1134-47-0; 66514-99-6; 69308-37-8; 70206-22-3; 63701-56-4; 63701-55-3 | NA |
| *3-(p-chlorophenyl)-4-hydroxybutyric acid* | / | Metabo lite |
| *Arbaclofen placarbil* | 847353-30-4 | Prodrug |

| *Mexiletine and related compounds* | | |
|---|---|---|
| *Mexiletine* | , 31828-71-4 ; 5370-01-4 | |
| *6-hydroxymethylmexiletine* | 53566-98-6 | Metabo lite |
| *4-hydroxymexiletine* | 53566-99-7 | Metabo lite |
| *3-hydroxymexiletine (MHM)* | 129417-37-4 | Metabo lite |
| *N-hydroxymexiletine glucuronide* | 151636-18-9 | Metabo lite |

| *Sulfisoxazole and related compounds* | | |
|---|---|---|
| *Sulfisoxazole* | 127-69-5 ; 4299-60-9 | |
| *N(4)-acetylsulfisoxazole* | 4206-74-0 | Metabo lite |
| *Sulfisoxazole acetyl* | 80-74-0 | Prodrug |
| *Sulfamethoxazole* | 723-46-6 | 0.52 |

| *Cinacalcet and related compounds* | | |
|---|---|---|
| *Cinacalcet* | 226256-56-0;364782-34-3 | |
| *Hydrocinnamic acid* | 501-52-0 | Metabo lite |

| *Torasemide and related compounds* | | |
|---|---|---|
| *Torasemide* | 56211-40-6;72810-59-4 | |
| *Hydroxytorasemide* | 99300-68-2; 99300-67-1 | Metabo lite |
| *Carboxytorasemide* | | Metabo lite |
| *Tolbutamide* | 64-77-7 | 0.55 |

More preferably, drug compositions of the invention may comprise 1, 2, 3, 4 or 5 distinct drugs, even more preferably 2, 3 or 4 distinct drugs for combinatorial treatment of epilepsy or a related disorder in a subject in need thereof. In a preferred embodiment, the drugs of the invention are used in combination(s) for combined, separate or sequential administration, in order to provide the most effective effect.

In a most preferred embodiment, compositions of this invention, for use in the treatment of epilepsy or related disorders, comprise the following drug combination for combined, separate or sequential administration:
- baclofen and acamprosate.

In another embodiment, the invention relates to a composition for use in the treatment of epilepsy or related disorders comprising at least acamprosate, or a salt, or sustained release formulation thereof.

This invention relates also to a composition comprising a combination of acamprosate and at least one compound selected from the group consisting of baclofen, cinacalcet, mexiletine, sulfisoxazole and torasemide, or salts or sustained release formulations thereof, for simultaneous, separate or sequential administration.

A further object of this invention resides in the use of a composition as defined above for the manufacture of a medicament for treating epilepsy or a related disorder.

As indicated previously, in a combination therapy of this invention, the compounds or drugs may be formulated together or separately, and administered together, separately or sequentially.

A further object of the invention is a composition for use in a method of treating epilepsy or a related disorder, the method comprising simultaneously, separately or sequentially administering to a subject in need thereof an effective amount of a composition as disclosed above.

In this regard, in a preferred embodiment, the invention relates to a composition for use in a method of treating epilepsy or a related disorder in a subject in need thereof, comprising administering simultaneously, separately or sequentially to the subject an effective amount of a combination of acamprosate and at least one compound selected from the group consisting of baclofen, cinacalcet, mexiletine, sulfisoxazole and torasemide.

In a more preferred embodiment, the invention relates to a composition for use in the method of treating epilepsy or a related disorder in a subject in need thereof, comprising administering simultaneously, separately or sequentially to the subject an effective amount of the following drug combination:
- baclofen and acamprosate.

The compositions of the invention typically comprise one or several pharmaceutically acceptable carriers or excipients. Also, for use in the present invention, the drugs or compounds are usually mixed with pharmaceutically acceptable excipients or carriers.

In this regard, a further object of this invention is a method of preparing a pharmaceutical composition, the method comprising mixing the above compounds or compound combinations in an appropriate excipient or carrier.

Although very effective *in vitro* and *in vivo,* depending on the subject or specific condition, the above methods, compositions or combination therapies may further be used in conjunction or association or combination with additional drugs or treatments.

Additional therapies used in conjunction with drug(s) or drug(s) combination(s) according to the present invention, may comprise one or more drug(s) that ameliorate symptoms of epilepsy disease, one or more drug(s) that could be used for palliative treatment of epilepsy disease or one or more drug(s) currently evaluated in the frame of clinical trials for treating of epilepsy disease.

Drugs or drug combinations of the invention can enhance or maintain the therapeutic effect of an already approved drug(s) treating convulsing aspect of the disorder. More particularly, combinations of the invention can allow an improvement of the treatment of pharmacoresistant epilepsy.

In this regard, a further disclosure relates to a composition, for use in the treatment of epilepsy or related disorders, comprising a composition as defined above, in combination with at least one compound selected from the group consisting of 4-aminopyridine, abatacept, carbamazepine, corticotropin, alpha-lipoic acid, amifampridine phosphate, aminobenzoate potassium, astaxanthin, beraprost sodium, clonazepam, creatine, creatinol phosphate, cyclosporin, dexamethasone, dextromethorphan hydrobromide, diflunisal, distigmine bromide, droxidopa, eculizumab, epalrestat, epoetin alfa, eslicarbazepine acetate, etanercept, fingolimod hydrochloride, ezogabine, gabapentin, gentamicin, glatiramer acetate, gosha-jinki-gan, human normal immunoglobulin, huperzine a, idebenone, interferon beta-la, lacosamide, lamotrigine, lenalidomide, levacecamine, levetiracetam, levocamitine acetyl, limaprost, mazindol, memantine, methotrexate (implant), minocycline hydrochloride, mycophenolate mofetil, nabiximols, nebivolol, nefazodone, neostigmine, nicotinamide, nitroglycerin, oxandrolone, oxazacort, oxcarbazepine, pentoxifylline, oxpentifylline, phenobarbital, phenytoin, prednisone, pregabalin, pyridostigmine bromide, rituximab, rufinamide, sildenafil citrate, tacrolimus, tadalafil, tofizopam, topiramate, capsaicin, ubidecarenone, valproate, venlafaxine and zoledronate, or salts or sustained release formulations thereof.

In a preferred embodiment, this invention relates to a composition, for use in the treatment of epilepsy or related disorders, comprising a composition as defined above, in combination with at least one compound selected from the group consisting of ezogabine, pregabalin, levetiracetam, lamotrigine, topiramate, valproate, rufinamide, gabapentin, carbamazepine, clonazepam, oxcarbazepine, phenobarbital and phenytoin, or salts or sustained release formulations thereof. In a more preferred embodiment, compositions of this invention, for use in the treatment of epilepsy or related disorders, comprise the following drug combination for combined, separate or sequential administration:
- baclofen and acamprosate,
in combination with at least one compound selected from the group consisting of ezogabine, pregabalin, levetiracetam, lamotrigine, topiramate, valproate, rufinamide, gabapentin, carbamazepine, clonazepam, oxcarbazepine, phenobarbital and phenytoin, or salts or sustained release formulations thereof. In an even more preferred embodiment, compositions of this invention, for use in the treatment of epilepsy or related disorders, comprise baclofen, acamprosate and at least one compound selected from the group consisting of ezogabine, pregabalin, levetiracetam, lamotrigine, topiramate, valproate, rufinamide, gabapentin, carbamazepine, clonazepam, oxcarbazepine, phenobarbital and phenytoin, or salts or sustained release formulations thereof.

A further object of the invention is a composition for use in a method of treating epilepsy or a related disorder, the method comprising simultaneously, separately or sequentially administering to a subject in need thereof an effective amount of a composition as disclosed above.

In another embodiment, compositions or combination therapies of the invention can be used in conjunction with surgical therapy for epilepsy disease such as anterior temporal lobectomy, resection of lesions (*e.g*. tumors, arteriovenous malformations,...), or removal of the front portion of the temporal lobe including the amygdala and hippocampus.

In this regard, the invention relates to a composition as defined above for use in the treatment of epilepsy or related disorders in a subject who has undergone or who will undergo surgical therapy for epilepsy.

Epileptogenesis can develop during a long time before appearance of convulsing seizure. Thus, the treatment of epilepsy before convulsing seizure appearance and in prevention thereof is essential in order to alter the progression and course of the disease.

Some subjects are particularly at risk of developing epilepsy. Subjects at risk of developing epilepsy often exhibit risk factors such as genetic predispositions, neonatal seizures, head injuries, status epilepticus, stroke and other vascular diseases, brain infections or inflammation (such as meningitis) or spinal cord inflammation.

In this regard, in a preferred embodiment, the above methods, compositions or combination therapies can be used for the prevention, prophylaxis or retardation of symptoms provoked by or of the causes of epilepsy disease in a subject who is at risk of developing epilepsy or convulsing seizures or symptoms associated with epilepsy disease.

In another embodiment, the invention relates to compositions as defined above for use in the treatment of epilepsy comorbidities in a subject suffering from epilepsy.

In a preferred embodiment, the invention relates to compositions as defined above for use in the treatment of memory impairment, depression or anxiety in a subject suffering from epilepsy.

In another embodiment, the invention relates to compositions as defined above for use in the treatment of idiopathic or symptomatic epilepsies.

Therapy according to the invention may be provided at home, the doctor's office, a clinic, a hospital's outpatient department, or a hospital, so that the doctor can observe the therapy's effects closely and make any adjustments that are needed.

The duration of the therapy depends on the stage of the disease being treated, age and condition of the patient, and how the patient responds to the treatment. The dosage, frequency and mode of administration of each component of the combination can be controlled independently. For example, one drug may be administered orally while the second drug may be administered intramuscularly. Combination therapy may be given in on-and-off cycles that include rest periods so that the patient's body has a chance to recovery from any as yet unforeseen side-effects. The drugs may also be formulated together such that one administration delivers all drugs.

The administration of each drug of the combination may be by any suitable means that results in a concentration of the drug that, combined with the other component, is able to ameliorate the patient condition or efficiently treat the disease or disorder.

While it is possible for the drugs of the combination to be administered as the pure chemical, it is preferable to present them as a pharmaceutical composition, also referred to in this context as pharmaceutical formulation. Possible compositions include those suitable for oral, rectal, topical (including transdermal, buccal and sublingual), or parenteral (including subcutaneous, intramuscular, intravenous and intradermal) administration.

More commonly these pharmaceutical formulations are prescribed to the patient in "patient packs" containing a number dosing units or other means for administration of metered unit doses for use during a distinct treatment period in a single package, usually a blister pack. Patient packs have an advantage over traditional prescriptions, where a pharmacist divides a patient's supply of a pharmaceutical from a bulk supply, in that the patient always has access to the package insert contained in the patient pack, normally missing in traditional prescriptions. The inclusion of a package insert has been shown to improve patient compliance with the physician's instructions. Thus, the invention further includes a pharmaceutical formulation, as herein before described, in combination with packaging material suitable for said formulations. In such a patient pack the intended use of a formulation for the combination treatment can be inferred by instructions, facilities, provisions, adaptations and/or other means to help using the formulation most suitably for the treatment. Such measures make a patient pack specifically suitable for and adapted for use for treatment with the combination of the present invention.

The drug may be contained, in any appropriate amount, in any suitable carrier substance. The drug may be present in an amount of up to 99% by weight of the total weight of the composition. The composition may be provided in a dosage form that is suitable for the oral, parenteral (*e.g*., intravenously, intramuscularly), rectal, cutaneous, nasal, vaginal, inhalant, skin (patch), or ocular administration route. Thus, the composition may be in the form of, *e.g.,* tablets, capsules, pills, powders, granulates, suspensions, emulsions, solutions, gels including hydrogels, pastes, ointments, creams, plasters, drenches, osmotic delivery devices, suppositories, enemas, injectables, implants, sprays, or aerosols.

The pharmaceutical compositions may be formulated according to conventional pharmaceutical practice (see, *e.g.,* Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York).

Pharmaceutical compositions according to the invention may be formulated to release the active drug substantially immediately upon administration or at any predetermined time or time period after administration.

The controlled release formulations include (i) formulations that create a substantially constant concentration of the drug within the body over an extended period of time; (ii) formulations that after a predetermined lag time create a substantially constant concentration of the drug within the body over an extended period of time; (iii) formulations that sustain drug action during a predetermined time period by maintaining a relatively, constant, effective drug level in the body with concomitant minimization of undesirable side effects associated with fluctuations in the plasma level of the active drug substance; (iv) formulations that localize drug action by, *e.g.,* spatial placement of a controlled release composition adjacent to or in the diseased tissue or organ; and (v) formulations that target drug action by using carriers or chemical derivatives to deliver the drug to a particular target cell type.

Administration of drugs in the form of a controlled release formulation is especially preferred in cases in which the drug has (i) a narrow therapeutic index (*i.e.,* the difference between the plasma concentration leading to harmful side effects or toxic reactions and the plasma concentration leading to a therapeutic effect is small; in general, the therapeutic index, TI, is defined as the ratio of median lethal dose (LD50) to median effective dose (ED50)); (ii) a narrow absorption window in the gastro-intestinal tract; or (iii) a very short biological half-life so that frequent dosing during a day is required in order to sustain the plasma level at a therapeutic level.

Any of a number of strategies can be pursued in order to obtain controlled release in which the rate of release outweighs the rate of metabolism of the drug in question. Controlled release may be obtained by appropriate selection of various formulation parameters and ingredients, including, *e.g.,* various types of controlled release compositions and coatings. Thus, the drug is formulated with appropriate excipients into a pharmaceutical composition that, upon administration, releases the drug in a controlled manner (single or multiple unit tablet or capsule compositions, oil solutions, suspensions, emulsions, microcapsules, microspheres, nanoparticles, patches, and liposomes).

### Solid dosage forms for oral use

Formulations for oral use include tablets containing the composition of the invention in a mixture with non-toxic pharmaceutically acceptable excipients. These excipients may be, for example, inert diluents or fillers (*e.g*., sucrose, microcrystalline cellulose, starches including potato starch, calcium carbonate, sodium chloride, calcium phosphate, calcium sulfate, or sodium phosphate); granulating and disintegrating agents (*e.g*., cellulose derivatives including microcrystalline cellulose, starches including potato starch, croscarmellose sodium, alginates, or alginic acid); binding agents (*e.g*., acacia, alginic acid, sodium alginate, gelatin, starch, pregelatinized starch, microcrystalline cellulose, carboxymethylcellulose sodium, methylcellulose, hydroxypropyl methylcellulose, ethylcellulose, polyvinylpyrrolidone, or polyethylene glycol); and lubricating agents, glidants, and antiadhesives (*e.g.*, stearic acid, silicas, or talc). Other pharmaceutically acceptable excipients can be colorants, flavoring agents, plasticizers, humectants, buffering agents, and the like.

The tablets may be uncoated or they may be coated by known techniques, optionally to delay disintegration and absorption in the gastrointestinal tract and thereby providing a sustained action over a longer period. The coating may be adapted to release the active drug substance in a predetermined pattern (*e.g*., in order to achieve a controlled release formulation) or it may be adapted not to release the active drug substance until after passage of the stomach (enteric coating). The coating may be a sugar coating, a film coating (*e.g.*, based on hydroxypropyl methylcellulose, methylcellulose, methyl hydroxyethylcellulose, hydroxypropylcellulose, carboxymethylcellulose, acrylate copolymers, polyethylene glycols and/or polyvinylpyrrolidone), or an enteric coating (*e.g*., based on methacrylic acid copolymer, cellulose acetate phthalate, hydroxypropyl methylcellulose phthalate, hydroxypropyl methylcellulose acetate succinate, polyvinyl acetate phthalate, shellac, and/or ethylcellulose). A time delay material such as, *e.g.,* glyceryl monostearate or glyceryl distearate may be employed.

The solid tablet compositions may include a coating adapted to protect the composition from unwanted chemical changes, (*e.g*., chemical degradation prior to the release of the active drug substance). The coating may be applied on the solid dosage form in a similar manner as that described in Encyclopedia of Pharmaceutical Technology.

Drugs may be mixed together in the tablet, or may be partitioned. For example, a first drug is contained on the inside of the tablet, and a second drug is on the outside, such that a substantial portion of the second drug is released prior to the release of the first drug. Formulations for oral use may also be presented as chewable tablets, or as hard gelatin capsules wherein the active ingredient is mixed with an inert solid diluent (*e.g*., potato starch, microcrystalline cellulose, calcium carbonate, calcium phosphate or kaolin), or as soft gelatin capsules wherein the active ingredient is mixed with water or an oil medium, for example, liquid paraffin, or olive oil. Powders and granulates may be prepared using the ingredients mentioned above under tablets and capsules in a conventional manner.

Controlled release compositions for oral use may, *e.g.,* be constructed to release the active drug by controlling the dissolution and/or the diffusion of the active drug substance.

Dissolution or diffusion controlled release can be achieved by appropriate coating of a tablet, capsule, pellet, or granulate formulation of drugs, or by incorporating the drug into an appropriate matrix. A controlled release coating may include one or more of the coating substances mentioned above and/or, *e.g.,* shellac, beeswax, glycowax, castor wax, carnauba wax, stearyl alcohol, glyceryl monostearate, glyceryl distearate, glycerol palmitostearate, ethylcellulose, acrylic resins, dl-polylactic acid, cellulose acetate butyrate, polyvinyl chloride, polyvinyl acetate, vinyl pyrrolidone, polyethylene, polymethacrylate, methylmethacrylate, 2-hydroxymethacrylate, methacrylate hydrogels, 1,3 butylene glycol, ethylene glycol methacrylate, and/or polyethylene glycols. In a controlled release matrix formulation, the matrix material may also include, *e.g.,* hydrated metylcellulose, carnauba wax and stearyl alcohol, carbopol 934, silicone, glyceryl tristearate, methyl acrylate-methyl methacrylate, polyvinyl chloride, polyethylene, and/or halogenated fluorocarbon.

A controlled release composition containing one or more of the drugs of the claimed combinations may also be in the form of a buoyant tablet or capsule (*i.e.,* a tablet or capsule that, upon oral administration, floats on top of the gastric content for a certain period of time). A buoyant tablet formulation of the drug(s) can be prepared by granulating a mixture of the drug(s) with excipients and 20-75% w/w of hydrocolloids, such as hydroxyethylcellulose, hydroxypropylcellulose, or hydroxypropylmethylcellulose. The obtained granules can then be compressed into tablets. On contact with the gastric juice, the tablet forms a substantially water-impermeable gel barrier around its surface. This gel barrier takes part in maintaining a density of less than one, thereby allowing the tablet to remain buoyant in the gastric juice.

### Liquids for oral administration

Powders, dispersible powders, or granules suitable for preparation of an aqueous suspension by addition of water are convenient dosage forms for oral administration. Formulation as a suspension provides the active ingredient in a mixture with a dispersing or wetting agent, suspending agent, and one or more preservatives. Suitable suspending agents are, for example, sodium carboxymethylcellulose, methylcellulose, sodium alginate, and the like.

### Parenteral compositions

The pharmaceutical composition may also be administered parenterally by injection, infusion or implantation (intravenous, intramuscular, subcutaneous, or the like) in dosage forms, formulations, or via suitable delivery devices or implants containing conventional, non-toxic pharmaceutically acceptable carriers and adjuvants. The formulation and preparation of such compositions are well known to those skilled in the art of pharmaceutical formulation.

Compositions for parenteral use may be provided in unit dosage forms (*e.g.,* in single-dose ampoules), or in vials containing several doses and in which a suitable preservative may be added (see below). The composition may be in form of a solution, a suspension, an emulsion, an infusion device, or a delivery device for implantation or it may be presented as a dry powder to be reconstituted with water or another suitable vehicle before use. Apart from the active drug(s), the composition may include suitable parenterally acceptable carriers and/or excipients. The active drug(s) may be incorporated into microspheres, microcapsules, nanoparticles, liposomes, or the like for controlled release. The composition may include suspending, solubilizing, stabilizing, pH-adjusting agents, and/or dispersing agents.

The pharmaceutical compositions according to the invention may be in the form suitable for sterile injection. To prepare such a composition, the suitable active drug(s) are dissolved or suspended in a parenterally acceptable liquid vehicle. Among acceptable vehicles and solvents that may be employed are water, water adjusted to a suitable pH by addition of an appropriate amount of hydrochloric acid, sodium hydroxide or a suitable buffer, 1,3-butanediol, Ringer's solution, and isotonic sodium chloride solution. The aqueous formulation may also contain one or more preservatives (*e.g*., methyl, ethyl or n-propyl p-hydroxybenzoate). In cases where one of the drugs is only sparingly or slightly soluble in water, a dissolution enhancing or solubilizing agent can be added, or the solvent may include 10-60% w/w of propylene glycol or the like.

Controlled release parenteral compositions may be in form of aqueous suspensions, microspheres, microcapsules, magnetic microspheres, oil solutions, oil suspensions, or emulsions. Alternatively, the active drug(s) may be incorporated in biocompatible carriers, liposomes, nanoparticles, implants, or infusion devices. Materials for use in the preparation of microspheres and/or microcapsules are, *e.g.,* biodegradable/bioerodible polymers such as polygalactin, poly-(isobutyl cyanoacrylate), poly(2-hydroxyethyl-L-glutamnine). Biocompatible carriers that may be used when formulating a controlled release parenteral formulation are carbohydrates (*e.g.,* dextrans), proteins (*e.g.,* albumin), lipoproteins, or antibodies. Materials for use in implants can be non-biodegradable (*e.g.*, polydimethyl siloxane) or biodegradable (*e.g*., poly(caprolactone), poly(glycolic acid) or poly(ortho esters)).

### Alternative routes

Although less preferred and less convenient, other administration routes, and therefore other formulations, may be contemplated. In this regard, for rectal application, suitable dosage forms for a composition include suppositories (emulsion or suspension type), and rectal gelatin capsules (solutions or suspensions). In a typical suppository formulation, the active drug(s) are combined with an appropriate pharmaceutically acceptable suppository base such as cocoa butter, esterified fatty acids, glycerinated gelatin, and various water-soluble or dispersible bases like polyethylene glycols. Various additives, enhancers, or surfactants may be incorporated.

The pharmaceutical compositions may also be administered topically on the skin for percutaneous absorption in dosage forms or formulations containing conventionally non-toxic pharmaceutical acceptable carriers and excipients including microspheres and liposomes. The formulations include creams, ointments, lotions, liniments, gels, hydrogels, solutions, suspensions, sticks, sprays, pastes, plasters, and other kinds of transdermal drug delivery systems. The pharmaceutically acceptable carriers or excipients may include emulsifying agents, antioxidants, buffering agents, preservatives, humectants, penetration enhancers, chelating agents, gel-forming agents, ointment bases, perfumes, and skin protective agents.

The preservatives, humectants, penetration enhancers may be parabens, such as methyl or propyl p-hydroxybenzoate, and benzalkonium chloride, glycerin, propylene glycol, urea, etc.

The pharmaceutical compositions described above for topical administration on the skin may also be used in connection with topical administration onto or close to the part of the body that is to be treated. The compositions may be adapted for direct application or for application by means of special drug delivery devices such as dressings or alternatively plasters, pads, sponges, strips, or other forms of suitable flexible material.

### Dosages and duration of the treatment

It will be appreciated that the drugs of the combination may be administered concomitantly, either in the same or different pharmaceutical formulation or sequentially. If there is sequential administration, the delay in administering the second (or additional) active ingredient should not be such as to lose the benefit of the efficacious effect of the combination of the active ingredients. A minimum requirement for a combination according to this description is that the combination should be intended for combined use with the benefit of the efficacious effect of the combination of the active ingredients. The intended use of a combination can be inferred by facilities, provisions, adaptations and/or other means to help using the combination according to the invention.

Therapeutically effective amounts of the drugs in a combination of this invention include, e.g., amounts that are effective for reducing epilepsy disease symptoms, halting or slowing the progression of the disease once it has become clinically manifest, or prevention or reduction of the risk of developing the disease.

Although the active drugs of the present invention may be administered in divided doses, for example two or three times daily, a single daily dose of each drug in the combination is preferred, with a single daily dose of all drugs in a single pharmaceutical composition (unit dosage form) being most preferred.

Administration can be one to several times daily for several days to several years, and may even be for the life of the patient. Chronic or at least periodically repeated long-term administration is indicated in most cases.

The term "unit dosage form" refers to physically discrete units (such as capsules, tablets, or loaded syringe cylinders) suitable as unitary dosages for human subjects, each unit containing a predetermined quantity of active material or materials calculated to produce the desired therapeutic effect, in association with the required pharmaceutical carrier.

The amount of each drug in a preferred unit dosage composition depends upon several factors including the administration method, the body weight and the age of the patient, the stage of the disease, the risk of potential side effects considering the general health status of the person to be treated. Additionally, pharmacogenomic (the effect of genotype on the pharmacokinetic, pharmacodynamic or efficacy profile of a therapeutic) information about a particular patient may affect the dosage used.

Except when responding to especially impairing cases, where higher dosages may be required, the preferred dosage of each drug in the combination will usually lie within the range of doses not above the dosage usually prescribed for long-term maintenance treatment or proven to be safe in phase 3 clinical studies.

One remarkable advantage of the invention is that each compound may be used at low doses in a combination therapy, while producing, in combination, a substantial clinical benefit to the patient. The combination therapy may indeed be effective at doses where the compounds have individually low or no effect. Accordingly, a particular advantage of the invention lies in the ability to use sub-optimal doses of each compound, *i.e.,* doses which are lower than therapeutic doses usually prescribed, preferably 1/2 of therapeutic doses, more preferably 1/3, 1/4, 1/5, or even more preferably 1/10 of therapeutic doses. In particular examples, doses as low as 1/20, 1/30, 1/50, 1/100, or even lower, of therapeutic doses are used.

At such sub-therapeutic dosages, the compounds would exhibit no side effect, while the combination(s) according to the invention are fully effective in treating epilepsy disease.

A preferred dosage corresponds to amounts from 1% up to 50% of those usually prescribed for long-term maintenance treatment.

The most preferred dosage may correspond to amounts from 1% up to 10% of those usually prescribed for long-term maintenance treatment.

Specific examples of dosages of drugs (Quantity equivalent to active molecule) for use in the invention are provided below:
- Acamprosate between 1 and 1000 mg per day, preferably less than 400 mg per day, more preferably less than 200 mg per day, such dosages being particularly suitable for oral administration.
- Baclofen between 0.01 to 150 mg per day, preferably less than 100 mg per day, more preferably less than 50 mg per day, such dosages being particularly suitable for oral administration.
- Mexiletine orally from about 6 to 120 mg per day, preferably less than 60 mg per day, more preferably less than 30 mg per day, such dosages being particularly suitable for oral administration,
- Torasemide orally from about 0.05 to 4 mg per day, preferably less than 2 mg per day, more preferably less than 1 mg per day, such dosages being particularly suitable for oral administration,
- Sulfisoxazole orally from about 20 to 800 mg per day, preferably less than 400 mg per day, more preferably less than 200 mg per day, such dosages being particularly suitable for oral administration,
- Cinacalcet orally from about 0.3 to 36 mg per day, preferably less than 20 mg per day, more preferably less than 10 mg per day, such dosages being particularly suitable for oral administration.

In combinations of the invention, the molar ratio between drugs may vary e.g., from 0.001 to 1000. Also, the ratio of the drug(s) and excipient in a composition of the invention advantageously vary between 0.001 and 1000.

When the composition comprises, as active ingredients, only baclofen and acamprosate, these two compounds may be used in different ratios, *e.g.,* at a weight ratio baclofen/acamprosate calcium comprised between 0.05 to 1000 (w/w), preferably between 0.05 to 500 (w/w), more preferably between 0.05 to 100 (w/w), more preferably between 0.05 to 50 (w/w).

It will be understood that the amount of the drug or the drug combination actually administered will be determined by a physician, in the light of the relevant circumstances including the condition or conditions to be treated, the exact composition to be administered, the age, weight, and response of the individual patient, the severity of the patient's symptoms, and the chosen route of administration. Therefore, the above dosage ranges are intended to provide general guidance and support for the teachings herein.

The following examples are given for purposes of illustration.

### EXAMPLES

*All animal procedures have been conducted in compliance to the existing legislation and regulations (Decree No. 87-848 of 19 October 1987: implemented in April 1988, incorporated Directive 86*/*609*/*EC into French law, amended by Decree No. 2001-464 of 29 May 2001 and by Decision of 20 June 2001).*

In order to demonstrate the efficacy of the drugs of this invention in the prevention from electrical and chemical induced seizures and in the improvement of the performances in memory tasks following electrically or chemically induced seizures, we tested rats or mice in three widely used behavioral tests and in *in vitro, ex vivo* and *in vivo* stimulation tests.

### I. In vitro efficacy of the drug combinations of the invention: drug combinations of the invention are efficient in preventing glutamate toxicity toward neuronal cells

Glutamate, the main excitatory neurotransmitter, is involved in the pathogenesis of epilepsy. On one hand, an abnormal amplification of glutamatergic activity is thought to be involved in the generation and spread of seizures. On the other hand, an excessive release of glutamate results in the overstimulation of the glutamate receptors and in the subsequent increase of an inflow of calcium leading to neuronal cell death (glutamate excitoxicity).

In this set of experiment, candidate compounds have been tested for their ability to prevent or reduce the toxic effects of glutamate toxicity on neuronal cells. The drugs were first tested individually, followed by assays of their combinatorial action.

### Neuronal cell preparation

The efficacy of drug combinations of the invention was assessed on primary cortical neuron cells.

Rat cortical neurons were cultured as described by Singer *et al.* [30]. Briefly, pregnant female rats of 15 days gestation were killed by cervical dislocation (Rats Wistar) and the fetuses were removed from the uterus. The cortex was removed and placed in ice-cold medium of Leibovitz (L15) containing 2% of penicillin 10.000 U/mL and streptomycin 10mg/mL and 1% of bovine serum albumin (BSA). Cortices were dissociated by trypsin for 20 min at 37°C (0.05%). The reaction was stopped by the addition of Dulbecco's modified Eagle's medium (DMEM) containing DNase1 grade II and 10% of foetal calf serum (FCS). Cells were then mechanically dissociated by 3 serial passages through a 10 mL pipette and centrifuged at 515 x g for 10 min at +4°C. The supernatant was discarded and the pellet of cells was resuspended in a defined culture medium consisting of Neurobasal supplemented with B27 (2%), L-glutamine (0.2 mM), 2% of PS solution and 10 ng/mL of BDNF. Viable cells were counted in a Neubauer cytometer using the trypan blue exclusion test. The cells were seeded at a density of 30 000 cells/well in 96 well-plates (wells were pre-coated with poly-L-lysine (10 µg/mL)) and were cultured at +37°C in a humidified air (95%)/CO2 (5%) atmosphere.

### Glutamate toxicity assays

The neuroprotective effect of compounds was assessed by quantification of the neurite network (Neurofilament immunostaining (NF)) which specifically reveals the glutamatergic neurons.

After 12 days of neuron culture, drugs of the candidate combinations were solved in culture medium (+0.1% DMSO). Candidate drugs or drug combinations were then pre-incubated with neurons for 1 h before the glutamate injury. Glutamate was added for 20 min, to a final concentration of 40 µM in presence of candidate drugs or drug combinations. At the end of the incubation, medium was changed with medium with candidate combination but without glutamate. The culture was fixed 24 h after glutamate injury. MK801 (Dizocilpinehydrogen maleate, 77086-22-7 - 20 µM) was used as a positive control.

After permeabilization with saponin (Sigma), cells were blocked for 2 h with PBS containing 10% goat serum, then the cells were incubated with mouse monoclonal primary antibody against Neurofilament antibody (NF, Sigma). This antibody was revealed with Alexa Fluor 488 goat anti-mouse IgG.

Nuclei of cells were labeled by a fluorescent marker (Hoechst solution, SIGMA), and neurite network quantified. Six wells per condition were used to assess neuronal survival in 3 different cultures.

### Results

All of the tested drug combinations give a protective effect against glutamate toxicity for cortical neuronal cells.

As exemplified in figures 1 to 4, combinations of the invention strongly protect neurons from glutamate toxicity under experimental conditions described above. It is noteworthy that an effective protection is noticed using drug concentrations at which drugs used alone have no significant or lower protective effect.

Indeed, as exemplified in figure 1 (reference), mexiletine+cinacalcet combination efficiently protects neuronal cells from glutamate toxicity, whereas no protection at all is afforded by the single drugs. Baclofen+acamprosate (fig. 3) combination gives also a protective effect against glutamate toxicity for cortical neuronal cells. Combination of baclofen and acamprosate induces an improvement of more than 200% compared to acamprosate alone and more than 47% compared to baclofen used alone. Improvement in protection afforded by the sulfisoxazole+torasemide combination is even more important when compared to the level of protection by either sulfisoxazole or torasemide alone (fig. 2) (reference). As shown in figure 4, (reference) the combination of baclofen and torasemide also provides a significant protection against glutamate toxicity, whereas at the same doses, torasemide and baclofen give no significant results (protection of 8% and 13%, respectively).

### II. Ex vivo study of the effect of the combination of acamprosate and baclofen in the frequency of epileptiform discharges induced by a zero-magnesium ACSF in rat hippocampal slices.

### Protocol

### Materials:

Experiments were carried out with 3 to 4 week-old Sprague-Dawley rats (from Elevage Janvier, FRANCE). The rats were sacrified by decapitation, without previous anesthesia. The brain was quickly removed and soaked in ice-cold oxygenated buffer with the following composition: KCl 2 mM, NaH₂PO₄ 1.2 mM, MgCl₂ 7 mM, CaCl₂ 0.5 mM, NaHCO₃ 26 mM, glucose 11 mM and saccharose 250 mM.

Hippocampal slices were prepared at 400 µm thickness with a McILWAIN tissue chopper.

Then, slices were incubated at room temperature for at least 1 h in Artificial Cerebro-Spinal Fluid (ACSF) of the following composition: NaCl 126 mM, KCl 3.5 mM, NaH₂PO₄ 1.2 mM, MgCl₂ 1.3 mM, CaCl₂ 2 mM, NaHCO₃ 25 mM and glucose 11 mM.

During experiments, slices were first perfused with oxygenated ACSF described above and then with a zero-magnesium/high-potassium ACSF, of the following composition: NaCl 126 mM, KCl 7 mM, NaH₂PO₄ 1.2 mM, MgCl₂ 0 mM, CaCl₂ 2 mM, NaHCO₃ 25 mM and glucose 11 mM.

### Compounds handling:

Acamprosate calcium (ref. A6981, Sigma, 10 mg) was prepared from powder, as a 10 mM stock solution in deionized water, aliquoted and stored at -20°C. On the day of experiments, one aliquot was thawed and intermediate stock solution was prepared in deionized water, to be finally diluted in the perfused ACSF. The final dilution in the ACSF that was perfused was performed within a 2 h time-window from the compound thawing.

Baclofen (ref. B5399, Sigma, 500 mg) was prepared from powder, as a 10 mM stock solution in deionized water, aliquoted and stored at -20°C. On the day of experiments, one aliquot was thawed and intermediate stock solution was prepared in deionized water, to be finally diluted in the perfused ACSF. The final dilution in the ACSF that was perfused was performed within a 2 h time-window from the compound thawing.

The H₂O concentration was adjusted to be equal to 0.3% in all the ACSF solutions.

### Perfusion:

During experiments, the slices were continuously perfused with the ACSF (bubbled with 95% O₂ - 5% CO₂) at the rate of 3 mL/min with a peristaltic pump (Multi-Electrode Array (MEA) chamber volume of about 1 mL). Complete solution exchange in the MEA chamber was achieved 20 s after the switch of solutions.

The perfusion liquid was continuously pre-heated at 37°C ± 0.1°C with a heating element located in the MEA amplifier headstage.

### Data acquisition and drug application:

### i) Recording set-up

All data were recorded with a MEA set-up commercially available from MultiChannel Systems (MCS) GmbH (Reutlinger, Germany) composed of a 4-channels stimulus generator and a 60-channels amplifier head stage connected to a 60-channels A/D card. Software for recordings and analysis was the one commercially available from MCS: MC Rack (4.5.0 release).

All of the experiments were carried out with a 3-dimensional MEA (Qwane, S.A., Lausanne, Switzerland) that consisted of 60 electrodes spaced by 200 µm.

### ii) Recording protocols and compounds applications

- A 400 µm thick rat hippocampal slice was placed on the MEA (200 µm distant electrodes) so that the multi-electrode grid covers the major surface of the slice.
- Drug combinations evaluations: following 5 min of control recording (to verify the absence of spontaneous activity), Epileptiform Discharges (ED) were triggered in hippocampal slices by a 30 min exposure to a zero-magnesium ACSF. Then, the compounds to evaluate were applied for a 60 min period (in zero-magnesium ACSF).

Control slices were recorded in parallel to compare the induction of ED in the presence or in the absence of the evaluated compounds: following 5 min of control recording, the occurrence of ED in a zero-magnesium ACSF was monitored for a 90 min period.

The ED frequency was monitored all along the experiment from MEA electrodes located in different sub-regions of the hippocampal slice.

Drug combinations were evaluated on 4 to 6 hippocampal slices from at least 3 different rats, recorded in parallel with control slices from the same rats.

A picture of the slice was taken at the beginning and at the end of the recording session to have an electronic picture of the slice and of the electrodes that were used for the recording as well as to verify that the slice has not moved above the electrodes during the recording session.

### Data analysis

The ED frequency (in Hz) was determined as following: the raw data were filtered with a Low Pass filter (Butterworth second order filter) set at 20 Hz. The ED number was monitored on-line (epileptiform event amplitudes had to be higher than 20 µV to be counted; a dead time of 200 ms was applied from each epileptiform event detected).

The amplitude of the events was monitored on-line over 1 s step using MCS software, and the ED amplitude was then determined off-line.

The ED frequency was averages by 30 s time slot for the all the electrodes inside one hippocampal slice. Data from each slice were averaged globally over the whole hippocampal area, and resulting data were pooled for experiments performed in same conditions. The frequency of ED for each condition was expressed as a function of time (± standard error of the mean: s.e.m.).

### Results

In control conditions, zero-magnesium ACSF has rapidly triggered ED which occurred with a frequency (rate) of about 0.4 Hz. Then, the ED rate has remained quite steady until the end of the experiments (fig. 5, grey line). Exposure to 1.6 nM acamprosate calcium applied in combination with 400 nM of baclofen at 35 min (dotted line) has decreased the rate of ED induced after exposure to a zero-magnesium ACSF (fig. 5, black line). Thus, the ED rate (0.39 ± 0.02 Hz at the end of the control period) was decreased after one-hour exposure to acamprosate calcium and baclofen (to 0.29 ± 0.05 Hz at endpoint).

Similar results are obtained for the other combinations of the invention (see table 2).

**Table 2 (reference)**

| **Combination** | **Epileptiform discharges in rat hippocampal slices** |
|---|---|
| Mexiletine + cinacalcet | improvement |
| Torasemide + sulfisoxazole | improvement |
| Torasemide + baclofen | improvement |

The compositions of the invention are thus effective in improving the protection against epileptic seizures.

### III. In vivo efficacy of the drugs and drug combinations of the invention

### A. Drugs alone are efficient in improving features of epilepsy in an in vivo model for epilepsy. Effect of acamprosate calcium on pentylenetetrazol (PTZ)-induced seizure in mice model.

### A.1 Protocol

### Test animals

Four to five week old male CD-1 mice of about 20g (Janvier; Le Genest St Isle - France) were acclimatized to the environment for one week before the experiment.

They were group-housed (5-10 mice per cage) and maintained in a room with controlled temperature (21-22°C) and a reversed light-dark cycle (12h/12h; lights on: 17:30 - 05:30; lights off: 05:30 - 17:30) with food and water available *ad libitum.*

Mice were randomly assigned to one of the different experimental groups.

### PTZ model

PTZ-induced seizures in mice are an accepted *in vivo* model for the screening of antiepileptic drugs. PTZ is a blocker of the chloride ionophore complex to the GABA_{A} receptor that has anticonvulsant effects after repeated or single dose administration and also affects several neurotransmitter systems, such as the GABAergic and glutamatergic systems. Seizures were induced by administration of 100 mg/kg (subcutaneously) of PTZ. Leviteracetam (500 mg/kg), used as an internal positive control, was administered 60 min before the PTZ injection. Drugs of the invention were injected *per os* 2 h before PTZ administration. Acamprosate calcium (two molecules of acamprosate for a calcium ion) was tested at three different doses.

As already stated, 100 mg/kg PTZ were injected subcutaneously and the animals were then observed for 1 h after injection. The latencies for clonic seizures, tonic seizures, and death were monitored. Clonic seizures consist in rhythmic contractions of forelimbs and/or hindlimbs. Tonic seizures consist in rigid extension of the forelimbs and/or hindlimbs.

### A.2 Calculation and statistical analysis

Analysis of variance (ANOVA) was performed on the result data. Fisher's Protected Least Significant Difference was used for pairwise comparisons. Statistical difference was assessed by Fisher's exact test. p values ≤ 0.05 were considered significant.

### A.3 Results

No visible sign of change in the health and behavior of mice was noticed before the administration of PTZ.

The administration of 100 mg/kg of PTZ is associated with significant appearance of clonic (fig. 6) and tonic seizures followed by animal death.

The PTZ-induced tonic seizure and death are reversed by the treatment with 500 mg/kg of levetiracetam that has contrarily no significant effect on time to clonic seizure (fig. 6, dark grey bar).

A significant increase in the latency to clonic seizure is observed for PTZ-treated mice receiving an injection of tested candidates drugs as illustrated for acamprosate calcium at 0.5 mg/kg (fig. 6, light grey bar). The dose of 0.032 mg/kg has also shown to increase the latency to clonic seizure (not shown).

The prevalence of mice with clonic seizure was reduced by 20% and 30% for the low and high doses of acamprosate calcium, respectively. The prevalence of mice with tonic seizure and the prevalence of death were reduced by 25% and 38% in the low and high doses of acamprosate calcium, respectively.

Acamprosate calcium is capable of interfering with PTZ-induced seizures, as reflected by the increase of the latency to clonic seizure. Acamprosate calcium also tended to reduce the prevalence of tonic seizures and the prevalence of death.

Hence drugs of the invention are efficient in correcting some features of induced seizures in the PTZ model.

### B. Effect of drug combinations as antiepileptic agents in in vivo models.

### B.1 Protocols-in vivo models for epilepsy

### Generalized chronic seizure in corneal kindled mouse model

Experiments are carried out according to Rowley *et al.,* 2010 [31].

**Animal husbandry:** Adult male CF1 mice weighing a minimum of 18 g are housed in a temperature-, humidity-, and light-controlled (12 h light:dark cycle) facility. Mice are group housed with permitted free access to food and water.

**Corneal kindling:** Mice are kindled according to the optimized protocol defined by Matagne and Klitgaard [32]. Briefly, mice are stimulated twice daily with a corneal stimulation of 3 mA (60 Hz) for 3 s for an average of 12 days. Prior to each stimulation, a drop of 0.9% saline containing 0.5% tetracaine hydrochloride is applied to the cornea to ensure local anesthesia and good electrical conductivity. Stimulations are delivered 4 h apart. Animals are considered kindled when they displayed five consecutive stage five seizures according to the Racine scale [33]. At the completion of the kindling acquisition, mice are permitted at least a 10-day stimulation-free period prior to any drug testing. Mice are stimulated once, a day before drug testing to ensure they had achieved and maintained a kindled state.

**Administration of compounds:** Drugs are freshly prepared just before each gavage or intraperitenally administration. From two days before evaluation and until the end of the experiment, drug combinations or the vehicle solution are administered twice daily (at 8:00 AM and 6:00 PM).

### Chronic seizures in kainate rat model

Experiments are carried out according to Drexel *et al.,* 2012 and Tchekalarova *et al.* 2011 [34,35].

**Animal husbandry:** Sixty-day-old male Wistar rats are habituated for 10 days and individually housed under standardized conditions. Food and water are available *ad libitum* throughout the study except during the tests.

**Administration of compounds:** Drugs are freshly prepared just before each gavage or ip administration. From two days before induction of status epilepticus (SE) with kainic acid (KA) and until the end of the experiment, drug combinations or the vehicle solution are administered twice daily (at 8:00 AM and 6:00 PM).

**Procedure for induction of status epilepticus with kainic acid:** Seizures are induced by injection with 10 mg/kg KA (5 mg/mL in saline, pH 7.0, intraperitonealy.). KA is diluted in sterile 0.9% saline at 2.5 mg/mL. Upon injection of KA, development of acute seizures and SE are monitored for at least 3 h for convulsive motor seizures.

**Motor seizure observation:** Upon KA treatment, experimental mice or rats are video monitored (24 h/day) for a period of 1 month. The following parameters are evaluated: latency to onset of the first spontaneous seizure and weekly seizure frequency. The severity of the seizure is also scored from III to V using an adapted Racine's scale. In addition to the video monitoring, all spontaneous motor seizures (SMS) detected during the routine and experimental manipulations of the animals are recorded. Video monitoring is performed with a light-sensitive black and white camera, and video recordings are visually analyzed.

### 6 Hz psychomotor seizure model

The 6 Hz psychomotor seizure model is used for the early identification of anticonvulsant activity of compounds particularly for compounds effective against therapy-resistant epilepsy.

Experiments are carried out according to Barton *et al.,* 2001 [37].

**Animal husbandry:** All experiments are performed on adult male Swiss mice weighing 22-26 g. The mice are kept in colony cages with free access to food and tap water under standardized housing conditions (natural light-dark cycle, temperature of 21±1 °C, relative humidity of 55±5%). After 7 days of adaptation to laboratory conditions, the animals are randomly assigned to experimental groups. All tests are performed between 9.00 AM and 3.00 PM.

**Administration of compounds:** Drugs are freshly prepared just before each gavage or intraperitoneal administration. From one day before initiation of psychomotor seizures evoked by 6 Hz corneal electrical stimulation and until the end of the experiment, drug combinations or the vehicle solution are administered twice daily (at 8:00 AM and 6:00 PM).

**Six-Hertz (6 Hz) seizure model:** Psychomotor (limbic) seizures are induced via an unique corneal stimulation (6 Hz, 0.2 ms rectangular pulse width, 32 mA, 3 s duration) delivered by an ECT Unit 5780. Ocular anesthetic (0.5% tetracaine) is applied to the mouse corneas 15 min before stimulation. The low frequency (6 Hz) long duration (3 s) seizures are characterized by immobility or stun, jaw and forelimb clonus, twitching of the vibrissae, and an elevated tail or Straub tail. Immediately following stimulation, mice are placed separately in Plexiglas cages (25 cm×15 cm×10 cm) for behavioral observation. Protection in the 6 Hz model is defined as the absence of a seizure.

### B̅.̅2̅ Results

Results show that drug combinations administration in both KA -treated rat and corneal kindled mice models significantly attenuates development of epileptiform activity and neuronal damage. Drug combinations of the invention delayed the onset of the first spontaneous seizure, weekly seizure frequency and decreased the "Motor seizure" score.

Drug combinations of the invention protect against the 6 Hz induced seizures. Furthermore, the use of drug combinations of the invention in addition to anticonvulsant drug enhances the activity of anticonvulsant.

### C. Effect of drug combinations as memory improvement in in vivo models.

Chronic epilepsy is coupled with cognitive deficits such as memory impairment. This is mainly due to a neuronal loss in the limbic areas (CA1, CA3, dentate gyrus of the hippocampus, amygdala and entorhinal cortex) [29].

### C.1 Protocols

Corneal kindled mice model and kainite rat model are used in these cognitive improvement experiments. Protocols for animal husbandry, administration of compounds, corneal kindling and procedure for induction of status epilepticus are described above.

Memory tasks are described below.

### Morris water maze

The Morris water maze [36], in which rodents learn to escape from water onto a hidden platform, is a widely used test of visual-spatial memory and hippocampal integrity. This apparatus consists of a 1.5-m-diameter tank with 10-cm-diameter platform submerged 1 cm below the surface of 21°C water obscured with black tempura paint (Prang, Heathrow, FL, USA). Rats or mice were trained to find the submerged platform. In this way, they were submitted to eight trials a day over 5 days. In this task, the position of the platform did not change but the start point varied at each trial.

During each training trial, the amount of time spent in each quadrant, distance travelled, and latency to target platform are measured. If the rodent did not find the target platform within 180 s (for rats) or 120 s (for mice), it is guided there and remained on the platform for 30 s. During the probe trials, the amount of time spent in each quadrant, distance traveled, time spent in the target zone (a smaller region approximately 13 cm in diameter around the target location), and number of platform crossings are recorded.

### Passive avoidance task

The rats injected with the respective drug or drugs are placed in an illuminated box (40×40×30 cm) connected to a dark box (rat: 40×40×30 cm / mice: 15×20×15 cm), which is equipped with an electric grid floor. Entrance to the dark box is punished by an electric footshock (rat: 0.7 mA for 2 s; mice: 0.3 mA for 3 s). The rodents that do not enter the dark compartment are excluded from the experiment. Twenty-four hours later, the same animals are put into the illuminated box and observed up to 180 s. Latency of entrance in the dark box is subsequently noted.

### C.2 Results

Rodents experimenting spontaneous motor seizures showed a higher cognitive impairment than control rodents both in the Morris water maze and in the passive avoidance task.

Drug combinations of the invention enhance performance in such behavioral tests as revealed by both the decreased latency during the acquisition phase and the probe trial recorded in the Morris water maze and by the decreased latency of entrance in the dark compartment, when compared to the vehicle-treated group.

### IV. Conclusion

The above examples show that compounds of the invention are efficient in the treatment of epileptic seizures and symptoms of epilepsy in *in vivo, ex vivo* and *in vitro* models for epilepsy.

### REFERENCES

1 Steinlein OK (2004) Genetic mechanisms that underlie epilepsy. Nature reviews. Neuroscience 5, 400-8.
2 Stafstrom CE (2006) Epilepsy: a review of selected clinical syndromes and advances in basic science. Journal of cerebral blood flow and metabolism : official journal of the International Society of Cerebral Blood Flow and Metabolism 26, 983-1004.
3 Vezzani A, Aronica E, Mazarati A & Pittman QJ (2013) Epilepsy and brain inflammation. Experimental neurology 244, 11-21.
4 Baker GA, Jacoby A, Buck D, Stalgis C & Monnet D (1997) Quality of life of people with epilepsy: a European study. Epilepsia 38, 353-62.
5 McCagh J, Fisk JE & Baker GA (2009) Epilepsy, psychosocial and cognitive functioning. Epilepsy research 86, 1-14.
6 Alessio A, Damasceno BP, Camargo CHP, Kobayashi E, Guerreiro CAM & Cendes F (2004) Differences in memory performance and other clinical characteristics in patients with mesial temporal lobe epilepsy with and without hippocampal atrophy. Epilepsy & behavior : E&B 5, 22-7.
7 Schmidt D & Rogawski MA (2002) New strategies for the identification of drugs to prevent the development or progression of epilepsy. Epilepsy research 50, 71-8.
8 Noachtar S & Borggraefe I (2009) Epilepsy surgery: a critical review. Epilepsy & behavior : E&B 15, 66-72.
9 Pitkänen A & Lukasiuk K (2009) Molecular and cellular basis of epileptogenesis in symptomatic epilepsy. Epilepsy & behavior: E&B 14 Suppl 1, 16-25.
10 Dudek FE & Staley KJ (2011) The time course of acquired epilepsy: implications for therapeutic intervention to suppress epileptogenesis. Neuroscience letters 497, 240-6.
11 Elger CE & Schmidt D (2008) Modern management of epilepsy: a practical approach. Epilepsy & behavior: E&B 12, 501-39.
12 Ettmayer P, Amidon GL, Clement B & Testa B (2004) Lessons learned from marketed and investigational prodrugs. Journal of medicinal chemistry 47, 2393-404.
13 Beaumont K, Webster R, Gardner I & Dack K (2003) Design of ester prodrugs to enhance oral absorption of poorly permeable compounds: challenges to the discovery scientist. Current drug metabolism 4, 461-85.
14 Heimbach T, Oh DM, Li LY, Rodriguez-Hornedo N, Garcia G & Fleisher D (2003) Enzyme-mediated precipitation of parent drugs from their phosphate prodrugs. International journal of pharmaceutics 261, 81-92.
15 Yang CY, Dantzig AH & Pidgeon C (1999) Intestinal peptide transport systems and oral drug availability. Pharmaceutical research 16, 1331-43.
16 Steffansen B, Nielsen CU, Brodin B, Eriksson AH, Andersen R & Frokjaer S (2004) Intestinal solute carriers: an overview of trends and strategies for improving oral drug absorption. European journal of pharmaceutical sciences: official journal of the European Federation for Pharmaceutical Sciences 21, 3-16.
17 Wermuth CG (2003) Designing prodrugs and bioprecusrors. In The Practice of Medicinal Chemistry (Hardbound, ed), 2nd ed., pp. 561-585. Academic Press.
18 Stella VJ (2007) Prodrugs: challenges and rewards. (A. Press and Springer, eds.) Springer Singapore Pte. Limited, New-York.
19 Pezron I, Mitra AK, Duvvuri S & Tirucherai GS (2002) Prodrug strategies in nasal drug delivery. Expert Opinion on Therapeutic Patents 12, 331-340.
20 Stella VJ (2004) Prodrugs as therapeutics. Expert Opinion on Therapeutic Patents 14, 277-280.
21 Stella VJ & Nti-Addae KW (2007) Prodrug strategies to overcome poor water solubility. Advanced drug delivery reviews 59, 677-94.
22 Higuchi T & Stella VJ (1975) Pro-drugs as Novel Drug Delivery System, ACS Sympos American Chemical Society, Washington, DC.
23 Roche EB (1977) Design of biopharmaceutical properties through prodrugs and analogs: a symposium, American P The Academy, Washington, DC.
24 Lal R, Sukbuntherng J, Tai EHL, Upadhyay S, Yao F, Warren MS, Luo W, Bu L, Nguyen S, Zamora J, Peng G, Dias T, Bao Y, Ludwikow M, Phan T, Scheuerman RA, Yan H, Gao M, Wu QQ, Annamalai T, Raillard SP, Koller K, Gallop MA & Cundy KC (2009) Arbaclofen placarbil, a novel R-baclofen prodrug: improved absorption, distribution, metabolism, and elimination properties compared with R-baclofen. The Journal of pharmacology and experimental therapeutics 330, 911-21.
25 Hanafi R, Mosad S, Abouzid K, Niess R & Spahn-Langguth H (2011) Baclofen ester and carbamate prodrug candidates: a simultaneous chromatographic assay, resolution optimized with DryLab. Journal of pharmaceutical and biomedical analysis 56, 569-76.
26 Leach AR & Gillet VJ An Introduction to Chemoinformatics (Springer-Verlag New York Inc, ed.).
27 Rahman SA, Bashton M, Holliday GL, Schrader R & Thornton JM (2009) Small Molecule Subgraph Detector (SMSD) toolkit. Journal of cheminformatics 1, 12.
28 Stahl H & Wermuth CG (2011) Pharmaceutical salts: Properties, selection, and use, 2nd ed. (Wiley-VCH, ed.).
29 Szyndler J, Piechal A, Blecharz-Klin K, Skórzewska A, Maciejak P, Walkowiak J, Turzyńska D, Bidziński A, Plaznik A & Widy-Tyszkiewicz E (2006) Effect of kindled seizures on rat behavior in water Morris maze test and amino acid concentrations in brain structures. Pharmacological reports : PR 58, 75-82.
30 Singer CA, Figueroa-Masot XA, Batchelor RH & Dorsa DM (1999) The mitogen-activated protein kinase pathway mediates estrogen neuroprotection after glutamate toxicity in primary cortical neurons. The Journal of neuroscience : the official journal of the Society for Neuroscience 19, 2455-63.
31 Rowley NM & White HS (2010) Comparative anticonvulsant efficacy in the corneal kindled mouse model of partial epilepsy: Correlation with other seizure and epilepsy models. Epilepsy research 92, 163-9.
32 Matagne A & Klitgaard H (1998) Validation of corneally kindled mice: a sensitive screening model for partial epilepsy in man. Epilepsy research 31, 59-71.
33 Racine RJ (1972) Modification of seizure activity by electrical stimulation: II. Motor seizure. Electroencephalography and Clinical Neurophysiology 32, 281-294.
34 Drexel M, Preidt AP & Sperk G (2012) Sequel of spontaneous seizures after kainic acid-induced status epilepticus and associated neuropathological changes in the subiculum and entorhinal cortex. Neuropharmacology 63, 806-17.
35 Tchekalarova J, Pechlivanova D, Atanasova T, Markova P, Lozanov V & Stoynev A (2011) Diurnal variations in depression-like behavior of Wistar and spontaneously hypertensive rats in the kainate model of temporal lobe epilepsy. Epilepsy & behavior: E&B 20, 277-85.
36 Morris R (1984) Developments of a water-maze procedure for studying spatial learning in the rat. Journal of neuroscience methods 11, 47-60.
37 Barton ME, Klein BD, Wolf HH & White HS (2001) Pharmacological characterization of the 6 Hz psychomotor seizure model of partial epilepsy. Epilepsy research 47, 217-27.

## Claims

1. A composition comprising at least acamprosate, or a salt or sustained release formulation thereof, for use in the treatment of epilepsy, benign Rolandic epilepsy, frontal lobe epilepsy, infantile spasms, myoclonic epilepsy, absence epilepsy, Lennox-Gastaut syndrome, Landau-Kleffner syndrome, Dravet syndrome, progressive myoclonus epilepsies, reflex epilepsy, Rasmussen's syndrome, temporal lobe epilepsy, limbic epilepsy, status epilepticus, abdominal epilepsy, massive bilateral myoclonus, catamenial epilepsy, Jacksonian seizure disorder, Lafora disease or photosensitive epilepsy in a subject in need thereof.

2. The composition for use of claim 1, further comprising at least one compound selected from baclofen, cinacalcet, mexiletine, sulfisoxazole or torasemide, or a salt or sustained release formulation thereof.

3. The composition for use of claim 1 or 2, wherein said composition comprises acamprosate and baclofen, or salt(s) or sustained release formulations thereof.

4. The composition for use of claim 1 or 2, further comprising at least one compound selected from ezogabine, pregabalin, levetiracetam, lamotrigine, topiramate, valproate, rufinamide, gabapentin, carbamazepine, clonazepam, oxcarbazepine, phenobarbital and phenytoin, or salt(s) or sustained release formulation(s) thereof.

5. The composition for use of any one of the preceding claims, which further comprises a pharmaceutically acceptable carrier or excipient.

6. The composition for use of any one of claims 2 to 5, wherein the compounds in said composition are formulated or administered together, separately or sequentially.

7. The composition for use of claim 6, wherein the compounds are formulated together.

8. The composition for use of any one of the preceding claims, wherein said composition is administered repeatedly to the subject.

9. The composition of any one of the preceding claims, for use in inhibiting epileptogenesis and epileptic seizure.

10. The composition for use of any one of the preceding claims, wherein said subject is at risk of developing epilepsy.

11. The composition for use of any one of the preceding claims, wherein said subject has undergone or will undergo surgical therapy for epilepsy.

12. The composition for use of any one of the preceding claims, wherein treating epilepsy comprises treating epilepsy comorbidities selected from memory impairment, cognitive decline, or anxiety, suicidal thoughts and/or depression in a subject in need thereof.

## Patentansprüche

1. Eine Zusammensetzung, umfassend wenigstens Acamprosat oder ein Salz oder eine Retardformulierung davon, zur Verwendung in der Behandlung von Epilepsie, benigner Rolando-Epilepsie, Stirnlappen-Epilepsie, kindlichen Spasmen, myoklonischer Epilepsie, Absence-Epilepsie, Lennox-Gastaut-Syndrom, Landau-Kleffner-Syndrom, Dravet-Syndrom, progressiver myoklonischer Epilepsien, Reflex-Epilepsie, Rasmussen-Syndrom, Temporallappen-Epilepsie, limbischer Epilepsie, Status epilepticus, abdominaler Epilepsie, massiver bilateraler myoklonischer katamenialer Epilepsie, Jackson-Anfall, Lafora-Syndrom oder photosensitiver Epilepsie in einer Person mit einem Bedarf daran.

2. Die Zusammensetzung zur Verwendung nach Anspruch 1, außerdem umfassend wenigstens eine Verbindung, ausgewählt aus Baclofen, Cinacalcet, Mexiletin, Sulfisoxazol oder Torasemid, oder ein Salz oder eine Retardformulierung davon.

3. Die Zusammensetzung zur Verwendung nach Anspruch 1 oder 2, wobei besagte Zusammensetzung Acamprosat und Baclofen oder Salz(e) oder Retardformulierungen davon umfasst.

4. Die Zusammensetzung zur Verwendung nach Anspruch 1 oder 2, außerdem umfassend wenigstens eine Verbindung, ausgewählt aus Ezogabin, Pregabalin, Levetiracetam, Lamotrigin, Topiramat, Valproat, Rufinamid, Gabapentin, Carbamazepin, Clonazepam, Oxcarbazepin, Phenobarbital und Phenytoin, oder Salz(e) oder Retardformulierung(en) davon.

5. Die Zusammensetzung zur Verwendung nach einem der vorhergehenden Ansprüche, die außerdem einen pharmazeutisch verträglichen Träger oder Exzipienten umfasst.

6. Die Zusammensetzung zur Verwendung nach einem der Ansprüche 2 bis 5, wobei die Verbindungen in besagter Zusammensetzung zusammen, getrennt oder aufeinanderfolgend formuliert oder verabreicht werden.

7. Die Zusammensetzung zur Verwendung nach Anspruch 6, wobei die Verbindungen zusammen formuliert werden.

8. Die Zusammensetzung zur Verwendung nach einem der vorhergehenden Ansprüche, wobei besagte Zusammensetzung der Person wiederholt verabreicht wird.

9. Die Zusammensetzung nach einem der vorhergehenden Ansprüche, zur Verwendung bei Hemmung von Epileptogenese und epileptischem Anfall.

10. Die Zusammensetzung zur Verwendung nach einem der vorhergehenden Ansprüche, wobei besagte Person ein Risiko zur Entwicklung von Epilepsie trägt.

11. Die Zusammensetzung zur Verwendung nach einem der vorhergehenden Ansprüche, wobei besagte Person sich einer chirurgischen Epilepsie-Therapie unterzogen hat oder unterziehen wird.

12. Die Zusammensetzung zur Verwendung nach einem der vorhergehenden Ansprüche, wobei die Behandlung von Epilepsie eine Behandlung von Epilepsie-Begleiterkrankungen, ausgewählt aus Gedächtnisschwund, kognitivem Verfall oder Angstgefühl, Suizidgedanken und/oder Depression, in einer Person mit einem Bedarf daran umfasst.

## Revendications

1. Composition comprenant au moins de l'acamprosate, ou un sel ou une formulation à libération prolongée de celui-ci, pour une utilisation dans le traitement de l'épilepsie, de l'épilepsie rolandique bénigne, de l'épilepsie du lobe frontal, des spasmes infantiles, de l'épilepsie myoclonique, de l'épilepsie-absences, du syndrome Lennox-Gastaut, du syndrome Landau-Kleffner, du syndrome Dravet, des épilepsies myocloniques progressives, de l'épilepsie réflexe, du syndrome de Rasmussen, de l'épilepsie du lobe temporal, de l'épilepsie limbique, du status epilepticus, de l'épilepsie abdominale, de la myoclonie massive bilatérale, de l'épilepsie cataméniale, du trouble convulsif de Jackson, de la maladie de Lafora ou de l'épilepsie photosensible chez un sujet qui en a besoin.

2. Composition pour une utilisation selon la revendication 1, comprenant en outre au moins un composé choisi parmi baclofène, cinacalcet, mexilétine, sulfisoxazole ou torasémide, ou un sel ou une formulation à libération prolongée de celui-ci.

3. Composition pour une utilisation selon les revendications 1 ou 2, ladite composition comprenant de l'acamprosate et du baclofène, ou des sels ou des formulations à libération prolongée de ceux-ci.

4. Composition pour une utilisation selon la revendication 1 ou 2, comprenant en outre au moins un composé choisi parmi ezogabine, prégabaline, lévétiracétam, lamotrigine, topiramate, valproate, rufinamide, gabapentine, carbamazépine, clonazépine, phénobarbital et phénytoïne, ou des sels ou des formulations à libération prolongée de ceux-ci.

5. Composition pour une utilisation selon l'une quelconque des revendications précédentes, qui comprend en outre un support ou excipient pharmaceutiquement acceptable.

6. Composition pour une utilisation selon l'une quelconque des revendications 2 à 5, dans laquelle les composés de ladite composition sont formulés ou administrés ensemble, séparément ou séquentiellement.

7. Composition pour une utilisation selon la revendication 6, dans laquelle les composés sont formulés ensemble.

8. Composition pour une utilisation selon l'une quelconque des revendications précédentes, ladite composition étant administrée de façon répétée au sujet.

9. Composition selon l'une quelconque des revendications précédentes, pour une utilisation dans l'inhibition de l'épileptogenèse et de la crise épileptique.

10. Composition pour une utilisation selon l'une quelconque des revendications précédentes, ledit sujet étant exposé au risque de développer l'épilepsie.

11. Composition pour une utilisation selon l'une quelconque des revendications précédentes, ledit sujet ayant subi ou pouvant subir une thérapie chirurgicale pour l'épilepsie.

12. Composition pour une utilisation selon l'une quelconque des revendications précédentes, ledit traitement de l'épilepsie comprenant le traitement des comorbidités épileptiques choisies parmi les troubles de la mémoire, le déclin cognitif, ou l'anxiété, des pensées suicidaires et/ou la dépression chez un sujet qui en a besoin.
